# EUROPÄISCHE PATENTANMELDUNG

(11) **EP 0 949 235 A2**
(43) Veröffentlichungstag der Anmeldung: **13.10.1999**
(21) Anmeldenummer: 99106421.3
(22) Anmeldetag: 29.03.1999
(51) Int. Cl.: C07C 43/13, C07C 41/16

(54) **Verfahren zur Herstellung von 3-Methoxy-1-propanol**

(30) Priorität: 07.04.1998 DE 19815634
(71) Anmelder: Bayer Aktiengesellschaft, 51368 Leverkusen (DE)
(72) Erfinder: Döbert, Frank Dr., 50933 Köln (DE); König, Bernd-Michael Dr., 51467 Bergisch Gladbach (DE); Wagner, Paul Dr., 40597 Düsseldorf (DE); Klausener, Alexander Dr., 50259 Pulheim (DE)

(57) **Zusammenfassung**

In vorteilhafter Weise wird 3-Methoxy-1-propanol hergestellt, indem man 1,3-Propandiol mit Methylchlorid in Gegenwart einer Base alkyliert.

## Beschreibung

Die vorliegende Erfindung betritt ein Verfahren zur Herstellung von 3-Methoxy-1-propanol aus 1,3-Propandiol.

3-Methoxy-1-propanol findet als Synthesebaustein zur Herstellung pharmazeutischer Wirkstoffe Verwendung. So kann man es beispielsweise bei der Synthese eines Magentherapeutikums verwenden (siehe EP-A 268 956).

Zur Herstellung von 3-Methoxy-1-propanol sind verschiedene Methoden bekannt, die jedoch alle nachteilig sind.

So wird in Can. J. Chem. 42, 2113 (1964) die Herstellung von 3-Methoxy-1-propanol aus 3-Chlor-1-propanol mit Natriummethanolat in Methanol beschrieben. Nach diesem Verfahren wird zu vorgelegtem Natriummethanolat in Methanol das 3-Chlor-1-propanol bei Siedetemperatur zugegeben. Die Ausbeute an 3-Methoxy-1-propanol beträgt nur 21 %. Die JP-OS 08-113546 beschreibt ebenfalls die Methoxylierung von 3-Chlor-1-propanol mit Natriummethanolat. Es soll jedoch eine Ausbeute von 69,8 % erzielt werden, was noch immer unbefriedigend ist.

Für die Umsetzung von 3-Brom-1-propanol mit Natriummethanolat wird nur eine Ausbeute von 20 % beschrieben (siehe Helv. Chim. Acta 63, 2152 (1980)).

Can. J. Chem. 63, 1833 (1985) beschreibt die Bildung von 3-Methoxy-1-propanol aus Natrium-3-hydroxypropylat mit Methyliodid, ebenso J. Amer. Chem. Soc. 65, 1276 (1943). Von Nachteil bei diesem Verfahren ist, daß teures Methyliodid als Reagenz verwendet und lediglich eine Ausbeute von 64 % erzielt wird.

Weiterhin ist die Herstellung von 3-Methoxy-1-propanol durch Reduktion von 3-Methoxypropionaldehyd (siehe DE 579 651 und Can. J. Chem. 63, 1833 (1985)), durch Hydratisierung von 3-Methoxypropen mit Quecksilberacetat und Natriumborhydrid (siehe J. Org. Chem. 46, 531 (1981)), durch Ringöffnung von 1,3-Dioxan mit Borhalogeniden (siehe J. Chem. Soc. Perkin. Trans. I, 1807 (1981)) und durch basische oder saure Ringöffnung von Oxetan mit Methanol (siehe J. Amer. Chem. Soc. 76, 56 (1954)) bekannt. Nachteilig bei diesen Methoden sind der Einsatz von teuren Reagenzien, geringen Ausbeuten und die zum Teil anfallenden giftigen Salze. Diese Verfahren sind also unwirtschaftlich und erfordern zum Teil besonderen ökologischen Aufwand.

Es besteht somit immer noch die Aufgabe, ein ökonomisch und ökologisch günstiges Verfahren zur Herstellung von 3-Methoxy-1-propanol aufzufinden, das von gut verfügbaren und preisgünstigen Ausgangsmaterialien ausgeht und gute Ausbeuten an gewünschtem Produkt liefert.

Es wurde nun ein Verfahren zur Herstellung von 3-Methoxy-1-propanol gefunden, das dadurch gekennzeichnet ist, daß man 1,3-Propandiol mit Methylchlorid in Gegenwart einer Base alkyliert. Gegebenenfalls können Lösungsmittel und/oder Hilfsstoffe, wie Katalysatoren, mit eingesetzt werden.

Das erfindungsgemäße Verfahren hat den Vorteil, daß 3-Methoxy-1-propanol direkt und auf einfache Weise aus preiswerten und gut verfügbaren Edukten ohne besonderen ökologischen Aufwand hergestellt werden kann. Überraschenderweise verläuft das erfindungsgemäße Verfahren sehr selektiv und führt zu unerwartet hohen Ausbeuten. Das Verfahren zeichnet sich somit dadurch aus, daß wenig Nebenprodukte gebildet werden und eine hervorragende Verwertung des Ausgangsmaterials 1,3-Propandiol gegeben ist.

Die erfindungsgemäße Umsetzung von 1,3-Propandiol mit Methylchlorid in Gegenwart einer Base kann grundsätzlich in allen unter den angewendeten Reaktionsbedingungen stabilen Lösungsmitteln durchgeführt werden. Vor allem eignen sich polare aprotische Lösungsmittel, z.B. Ketone wie Aceton, Diethylketon, Diisopropylketon und Methylethylketon, Nitrile wie Acetonitril, Amide wie Dimethylformamid, Dimethylacetatamid und N-Methyl-pyrrolidon, Sulfoxide wie Dimethylsulfoxid, Sulfone wie Sulfolan, Hexamethylphosphorsäuretriamid, Ether wie Diethylether, Diisopropylether, Tetrahydrofuran, 1,4-Dioxan, t-Butylmethylether, Ethylenglykoldimethylether und 1,3-Dimethoxypropan und unter den Reaktionsbedingungen flüssige Acetale wie Formaldehyddimethylacetal, 2,2-Dimethoxypropan, 1,3-Dioxolan und 1,3-Dioxan. Es können auch beispielsweise C₆-C₁₀-Aliphaten wie n-Hexan, n-Heptan, n-Oktan, i-Octan und n-Nonan und gegebenenfalls substituierte C₆- bis C₁₀-Aromaten wie Benzol, Toluol, o-, m- und p-Kresol, Chlorbenzole, Fluorbenzole und Dichlorbenzole als Lösungsmittel verwendet werden. Die Lösungsmittel lassen sich jeweils alleine oder in beliebigen Mischungen untereinander einsetzen.

In einer bevorzugten Ausführungsform wird 1,3-Propandiol im Überschuß eingesetzt, so daß dieses sowohl als Reaktionspartner als auch als Lösungsmittel fungiert.

Unabhängig von der Gegenwart von Lösungsmitteln ist das Verhältnis der Stoffmenge des 1,3-Propandiols, welches bei der Reaktion nicht umgesetzt wird, zu der Stoffmenge des 1,3-Propandiols, welches bei der Reaktion verbraucht wird, über einen weiteren Bereich variierbar. Es liegt üblicherweise zwischen 50:1 und 0,1:1, bevorzugt zwischen 10:1 und 0,5:1, besonders bevorzugt zwischen 5:1 und 1:1.

Als Basen können insbesondere solche mit einer hohen Protonenaffinität verwendet werden, die unter den angewendeten Reaktionsbedingungen hinreichend stabil und geeignet sind, alkoholische Hydroxylgruppen zu deprotonieren.

Zum Beispiel eignen sich Alkalimetall- und Erdalkalimetallhydroxide, -hydrogencarbonate und -carbonate, metallorganische Verbindungen wie Alkyllithium-, Aryllithium- und Alkylzinkverbindungen und Grignardverbindungen sowie höhermolekulare organische Stickstoffbasen wie 1,4-Diazabicyclo[2.2.2]octan (DABCO) oder 1,8-Diazabicyclo[5.4.0]undec-7-en (DBU). Bevorzugt sind Alkali- und Erdalkalihydroxide; besonders bevorzugt ist Natrium- und Kaliumhydroxid. Die jeweilige Base kann dem Reaktionsgemisch z.B. in fester, gelöster oder suspendierter Form zugesetzt werden. Im Falle der Verwendung von Alkalimetall- oder Erdalkalimetallhydroxiden kann es von besonderem Vorteil sein, diese Basen als wäßrige Lösungen in das Reaktionsgemisch einzutragen. Solche wäßrigen Alkalimetall- oder Erdalkalimetallhydroxidlösungen können eine Konzentration von größer als 1 Gew.-%, bevorzugt von größer als 10 Gew.-% und besonders bevorzugt eine Konzentration von größer als 30 Gew.-% aufweisen. Das Stoffmengenverhältnis der zugesetzten Base zu 1,3-Propandiol kann z.B. zwischen 1:0,5 und 1:50, bevorzugt zwischen 1:1 und 1:20, besonders bevorzugt zwischen 1:1 und 1:5 liegen.

Die erfindungsgemäße Umsetzung von 1,3-Propandiol mit Methylchlorid in Gegenwart einer Base kann grundsätzlich diskontinuierlich oder kontinuierlich durchgeführt werden.

In einer bevorzugten diskontinuierlichen Ausführungsform erfolgt die Durchführung des erfindungsgemäßen Verfahrens in einem sog. Semibatch-Verfahren, bei dem Methylchlorid gasförmig und Base in flüssiger Form simultan zu gegebenenfalls mit weiterem Lösungsmittel vermischtem 1,3-Propandiol zudosiert werden.

In einer bevorzugten kontinuierlichen Ausführungsform erfolgt die Zudosierung von Methylchlorid und Base kontinuierlich zu einem Stoffstrom von 1,3-Propandiol.

Unabhängig von der Art der Durchführung des erfindungsgemäßen Verfahrens können Methylchlorid und Base gleichzeitig oder nacheinander an einem gemeinsamen Ort oder räumlich getrennt voneinander zugegeben werden. Erfolgt die Zugabe an verschiedenen Orten oder zeitlich gegeneinander versetzt, so ist es zweckmäßig, zuerst die Base und anschließend das Methylchlorid einzudosieren.

Wird das erfindungsgemäße Verfahren auf diskontinuierliche Weise, beispielsweise in einem Semibatch-Verfahren, durchgeführt, so eignet sich hierfür als Reaktionsapparat im allgemeinen ein üblicher, diskontinuierlich betriebener Rührkesselreaktor. Bevorzugt ist ein solcher Rührkesselreaktor mit Rührelementen ausgestattet, die einen guten Eintrag des gasförmigen Methylchlorids in das Reaktionsmedium gewährleisten, wie Begasungsrührer, Zahnscheibenrührer, MIG-Rührer oder anderen dem Fachmann bekannten Begasungsrührer. Die Zugabe von Methylchlorid erfolgt dann durch solche mit Einrichtungen zur Begasung versehenen Rührelementen.

Wird das erfindungsgemäße Verfahren auf kontinuierliche Weise durchgeführt, so eignen sich als Reaktionsapparate hierfür beispielsweise übliche, kontinuierlich betriebene Rührkesselreaktoren, Rührkesselkaskaden oder verschiedene Arten von Rohrreaktoren wie Blasensäulenreaktoren, Strömungsrohrreaktoren, Schlaufenreaktoren oder andere dem Fachmann bekannte, zur kontinuierlichen Reaktionsdruchführung geeignete Systeme.

In einer besonders bevorzugten Variante des erfindungsgemäßen Verfahrens wird 1,3-Propandiol zunächst mit einem gegebenenfalls in Wasser gelösten Alkali- oder Erdalkalimetallhydroxid zum Umsatz gebracht und das entstandene Reaktionswasser sowie gegebenenfalls ins Reaktionsgemisch eingetragene Wasser vor der Zugabe von Methylchlorid aus dem Reaktionsgemisch entfernt. Diese Entwässerung ist z.B. durch eine einfache Destillation möglich, die bei Normaldruck oder bei vermindertem Druck betrieben werden kann. Schonender kann das Wasser durch eine Azeotropdestillation abgetrennt werden. Als Azeotropbildner eignen sich Substanzen, die mit Wasser ein Heteroazeotrop bilden können, wie 2-Methoxyethanol, Dimethoxymethan, Trimethylamin, 1-Butin-3-on, 3-Buten-nitril, Methacrylonitril, Dioxen, Butyronitril, 2-Butanon, Ethylvinylether, Tetrahydrofuran, Dioxan, Ethylacetat, Methylpropionat, Propylformiat, Diethylether, Methylpropylether, 1,1-Dimethoxyethan, 1,2-Dimethoxyethan, 2-Ethoxyethanol, Isopropylformiat, Ethoxymethoxymethan, 1-Methoxy-2-propanol, 2-Methoxy-1-propanol, 3-Methoxy-1-propanol, Furfural, 2-Methylfuran, Allylvinylether, Cyclopentanon, 1-Methoxy-1,3-butadien, 1,3-Dimethoxypropan, 2-Methyl-3-butin-2-ol, Allylacetat, 2,3-Pentandion, 2,4-Pentandion, Vinylpropionat, Cyclopentanol, Isopropenylethylether, Isopropylvinylether, 2-Pentanon, 3-Pentanon, Propylvinylether, Butylformiat, Propylacetat, 2-Methoxyethylacetat, Methoxymethylpropionat, n-Amylalkohol, tert.-Amylalkohol, tert.-Isobutylmethylether, Ethylpropylether, Isoamylalkohol, 3-Methylbutanol, 2-Pentanol, 3-Pentanol, 1,2-Dimethoxypropan, 1-Ethoxy-2-propanol, 1,1,2-Trimethoxyethan, Cyclohexen, 2-Ethyl-1,3-butadien, 1-Hexen-5-on, Cyclohexan, 1-Hexen, 4-Methyl-2-penten, Cyclohexanol, 2-Hexanon, 3-Hexanon, 4-Methyl-2-pentanon, Amylformiat, Butylacetat, Hexan, tert.-Amylmethylether, Butylethylether, Diiospropylether, aromatische Kohlenwasserstoffe und dem Fachmann bekannte sonstige Stoffe, die mit Wasser ein Azeotrop zu bilden vermögen. In einer bevorzugten Variante der Azeotropdestillation wird Toluol oder Benzol zum Ausschleusen des Wassers eingesetzt.

Die Wasserentfernung kann absatzweise oder kontinuierlich durchgeführt werden.

Die entwässerte Reaktionslösung wird in dieser bevorzugten Variante des erfindungsgemäßen Verfahrens dann mit Methylchlorid umgesetzt.

Zur Beschleunigung der erfindungsgemäßen Methylierungsreaktion können dem Reaktionsgemisch gegebenenfalls Brom- oder Jodsalze als Katalysatoren zugesetzt werden. Hierzu eignen sich besonders Alkali- und Erdalkalimetallbromide und -iodide. Vorzugsweise werden dem Reaktionsgemisch katalytische Mengen Natriumiodid oder Kaliumiodid zugesetzt. Solche Salze können z.B. in Mengen von 0,001 bis 10 Gew.-%, bevorzugt von 0,01 bis 5 Gew.-%, besonders bevorzugt von 0,1 bis 2 Gew.-%, jeweils bezogen auf das gesamte Reaktionsgemisch, eingesetzt werden.

Zur Erzielung hoher Raum-Zeit-Ausbeuten ist es vorteilhaft, das erfindungsgemäße Verfahren in einem Temperaturbereich zwischen 50 und 200°C, bevorzugt zwischen 70 und 160°C und besonders bevorzugt zwischen 80 und 150°C durchzuführen.

Das Methylchlorid kann dem Reaktionsgemisch gasförmig oder in verflüssigter Form zugegeben werden, wobei die Verflüssigung durch Druck und/oder Kühlung erreicht werden kann. Methylchlorid wird vorzugsweise gasförmig dem Reaktionsgemisch zugeführt. Der Partialdruck des Methylchlorids kann dabei z.B. zwischen 0,01 und 10 bar, bevorzugt zwischen 0,1 und 5 bar, besonders bevorzugt zwischen 0,5 und 4 bar, betragen. Der Methylchloriddruck kann mit dem Fortschritt der Umsetzung variiert werden. So kann es günstig sein, den Druck zum Ende der Reaktion hin anzuheben.

Die erfindungsgemäße Umsetzung kann z.B. mit Verweilzeiten zwischen 1 sec und 10 h, bevorzugt zwischen 1 min und 5 h, besonders bevorzugt zwischen 10 min und 4 h, durchgeführt werden.

Der aktuelle Druck im Reaktionsapparat ist abhängig von der Reaktionsrate. In der Regel beträgt der Gesamtdruck während der Reaktion 0,01 bis 50 bar, bevorzugt 0,1 bis 10 bar, besonders bevorzugt 0,5 bis 5 bar.

Das Molverhältnis von Methylchlorid zu Base kann z.B. zwischen 0,1 und 10, bevorzugt zwischen 0,5 und 5, besonders bevorzugt zwischen 0,8 und 1,2, liegen.

Die Aufarbeitung des Reaktionsgemisches kann auf im Prinzip bekannte Weise erfolgen. Beispielsweise kann man zunächst das während der Umsetzung aus der Base gebildete Salz abtrennen, etwa durch Filtration. Eine solche Filtration kann in üblichen, zur Feststoffabtrennung geeigneten Apparaten erfolgen, beispielsweise in Filternutschen, Bandfiltern, Blattfiltern, Kerzenfiltern, Filterpressen, Drehfiltern, Drucktrommelfiltern und Rotationsdruckfiltern. Man kann das Salz auch durch Zentrifugieren abtrennen. Das Filtrat enthält im wesentlichen nicht umgesetztes und gegebenenfalls als Lösungsmittel verwendetes 1,3-Propandiol, gegebenenfalls vorhandenes weiteres Lösungsmittel, gegebenenfalls vorhandene überschüssige Base, das hergestellte 3-Methoxy-1-propanol sowie einen im allgemeinen sehr geringen Anteil an 1,3-Dimethoxypropan. Dieses Gemisch läßt sich auf dem Fachmann bekannte Weise, bevorzugt destillativ, in die einzelnen Komponenten trennen. Abgetrenntes 1,3-Propandiol kann zur weiteren Umsetzung zurückgeführt werden.

Vorteilhaft beim erfindungsgemäßen Verfahren ist insbesondere die hohe Selektivität der Umsetzung der Reaktanden. Die erzielbare Selektivität der Bildung von 3-Methoxy-1-propanol ist i.a. größer als 85 %, häufig größer als 90 % und kann auch größer als 95 % sein.

### Beispiele

### Beispiel 1

In einem 1 l-Mehrhalskolben wurden 315 g 1,3-Propandiol vorgelegt. Es wurden 224 g einer 50 Gew.-%igen wäßrigen Kaliumhydroxid-Lösung zudosiert. Das entstandene Gemisch wurde unter vermindertem Druck wasserfrei destilliert. Man erhielt auf diese Weise 388 g einer Lösung des Monokaliumalkoholates von 1,3-Propandiol.

311 g der so hergestellten Lösung wurden in einem Autoklaven zusammen mit 3 g Kaliumiodid unter Stickstoff vorgelegt. Das Gemisch wurde auf 120°C aufgeheizt und mit Methylchlorid versetzt, das unter einem Druck von 2 bar während 1,7 Stunden dem Reaktor zugeführt wurde. Die Lösung wurde dann noch 1 Stunde lang bei 120°C nachgerührt. Es wurden insgesamt 84 g Methylchlorid aufgenommen.

Das Reaktionsprodukt enthielt gemäß gaschromatographischer Analyse nach Abkühlung und Abtrennung des ausgefallenen Kaliumchlorids durch Filtration 55 % 1,3-Propandiol, 38 % 3-Methoxy-1-propanol und 5 % Dimethoxypropan. Die Selektivität der Bildung von 3-Methoxy-1-propanol betrug somit 89 % und seine Ausbeute 71 % der Theorie, jeweils bezogen auf das eingesetzte Monokaliumsalz von 1,3-Propandiol.

### Beispiel 2

a) In einem 6 l-Mehrhalskolben wurden 4560 g 1,3-Propandiol zusammen mit 495,3 g gepulvertem Kaliumhydroxid (85 Gew.-%ig) vorgelegt. Es wurden 832 g einer 50 gew.-%igen wäßrigen Kaliumhydroxid-Lösung zudosiert. Das entstandene Gemisch wurde mit 200 ml Toluol durch Azeotropdestillation wasserfrei destilliert. Man erhielt auf diese Weise 5189 g einer Lösung des Monokaliumsalzes von 1,3-Propandiol.
b) 2060 g der so hergestellten Lösung wurden in einem Autoklaven zusammen mit 20 g Kaliumiodid unter Stickstoff vorgelegt. Das Gemisch wurde auf 100°C aufgeheizt und mit Methylchlorid versetzt, das unter einem Druck von 2 bar in 2 Stunden dem Reaktor zugeführt wurde. Es wurden insgesamt 302 g Methylchlorid aufgenommen. Das Reaktionsprodukt, nach Abkühlung und Abtrennung des ausgefallenen Kaliumchlorids mittels Filtration, enthielt gemäß gaschromatographischer Analyse 73 % 1,3-Propandiol, 21 % 3-Methoxypropanol und 1 % Dimethoxypropan. Die Selektivität der Bildung von 3-Methoxy-1-propanol betrug somit 96 % und seine Ausbeute 85 % der Theorie, jeweils bezogen auf das eingesetzte Monokaliumsalz von 1,3-Propandiol.

### Beispiel 3

256 g einer gemäß Beispiel 2 a) erhaltenen Lösung des Monokaliumsalzes von 1,3-Propandiol wurden in einem Autoklaven unter Stickstoff vorgelegt. Das Gemisch wurde auf 100°C aufgeheizt und mit Methylchlorid versetzt, das zunächst unter einem Druck von 2 bar während 1,3 Stunden und anschließend unter einem Druck von 3 bar während 1,3 Stunden dem Reaktor zugeführt wurde.

Es wurden insgesamt 38 g Methylchlorid aufgenommen. Das Reaktionsprodukt, nach Abkühlung und Abtrennung des ausgefallenen Kaliumchlorids durch Filtration, enthielt gemäß gaschromatographischer Analyse 74 % 1,3-Propandiol, 23 % 3-Methoxy-1-propanol und 2 % Dimethoxypropan. Die Selektivität der Bildung von 3-Methoxy-1-propanol betrug somit 93 % und seine Ausbeute 82 % der Theorie, jeweils bezogen auf das eingesetzte Monokaliumsalz von 1,3-Propandiol.

## Patentansprüche

1. Verfahren zur Herstellung von 3-Methoxy-1-propanol, dadurch gekennzeichnet, daß man 1,3-Propandiol mit Methylchlorid in Gegenwart einer Base alkyliert.

2. Verfahren nach Anspruch 1, dadurch gekennzeichnet, daß man in Gegenwart von Ketonen, Nitrilen, Amiden, Sulfoxiden, Sulfonen, Hexamethylphosphorsäuretriamid, Ethern, unter Reaktionsbedingungen flüssigen Acetalen, C₆-C₁₀-Aliphaten und/oder gegebenenfalls substituierten C₆-C₁₀-Aromaten als Lösungsmitteln arbeitet.

3. Verfahren nach Anspruch 1, dadurch gekennzeichnet, daß man 1,3-Propandiol im Überschuß einsetzt.

4. Verfahren nach Ansprüchen 1 bis 3, dadurch gekennzeichnet, daß man als Basen solche verwendet, die unter den Reaktionsbedingungen hinreichend stabil und geeignet sind, um alkoholische Hydroxidgruppen zu deprotonieren.

5. Verfahren nach Ansprüchen 1 bis 4, dadurch gekennzeichnet, daß das Molverhältnis der zugesetzten Base zu 1,3-Propandiol zwischen 1:0,5 und 1:50 liegt.

6. Verfahren nach Ansprüchen 1 bis 5, dadurch gekennzeichnet, daß man es diskontinuierlich durchführt.

7. Verfahren nach Ansprüchen 1 bis 5, dadurch gekennzeichnet, daß man es kontinuierlich durchführt.

8. Verfahren nach Ansprüchen 1 bis 7, dadurch gekennzeichnet, daß man 1,3-Propandiol zunächst mit Alkali- oder Erdalkalimetallhydroxid zum Umsatz bringt und das entstandene Reaktionswasser sowie gegebenenfalls ins Reaktionsgemisch eingetragene Wasser vor der Zugabe von Methylchlorid aus dem Reaktionsgemisch entfernt.

9. Verfahren nach Ansprüchen 1 bis 8, dadurch gekennzeichnet, daß man dem Reaktionsgemisch Brom- oder Iodsalze als Katalysatoren zusetzt.

10. Verfahren nach Ansprüchen 1 bis 9, dadurch gekennzeichnet, daß man es bei Temperaturen im Bereich 50 bis 200°C, Methylchlorid-Partialdrucken im Bereich 0,01 bis 10 bar und Gesamtdrucken im Bereich 0,01 bis 50 bar durchführt.

11. Verfahren nach Ansprüchen 1 bis 10, dadurch gekennzeichnet, daß 1,3-Propandiol als Lösungsmittel eingesetzt wird.
